# EUROPEAN PATENT APPLICATION

(11) **EP 4 740 854 A1**
(43) Date of publication of application: **13.05.2026**
(21) Application number: 23945187.5
(22) Date of filing: 07.07.2023
(51) Int. Cl.: A61B 5/145, A61B 5/1455, A61B 5/00

(54) **APPARATUS AND METHOD FOR SOBRIETY TEST**

(71) Applicant: LG Electronics Inc., Yeongdeungpo-gu Seoul 07336 (KR)
(72) Inventor: RHEE, Byungjoon, Seoul 06772 (KR)
(74) Representative: Schott, Jakob Valentin
(86) International application number: PCT/KR2023/009671
(87) International publication number: WO 2025/013958

(57) **Abstract**

Disclosed is an apparatus and method for a sobriety test. An apparatus for detecting an ethanol component in blood, according to at least one of various embodiments of the present disclosure, may comprise: a first sensor configured to emit a plurality of rays of light having different wavelengths to blood vessels of a target user; a light-receiving unit for receiving the light emitted by the first sensor to the blood vessels; and a processor for obtaining first data obtained by emitting light of a first wavelength and second data obtained by emitting light of a second wavelength, correcting the second data on the basis of the first data, and detecting ethanol component data in blood on the basis of the corrected second data.

## Description

### [Technical Field]

The present disclosure relates to an apparatus and method for drinking.

### [Background Art]

Various incidents and accidents caused by drinking continue to occur. Despite these incidents and accidents, most laws and common sense emphasize appropriate measures and countermeasures for drinking, rather than prohibiting drinking itself.

Most existing systems for measuring alcohol intake have adopted a method of analyzing alcohol (i.e., ethanol) in breath to determine whether a person is intoxicated.

These methods for measuring alcohol intake include semiconductor, electrochemical, and infrared (IR) methods.

Semiconductor methods measure alcohol intake by measuring the amount of electricity generated when alcohol components collide with a heated semiconductor, thereby analyzing alcohol temperature. While semiconductor methods offer the advantages of being inexpensive and portable, they suffer from low accuracy.

The most commonly used electrochemical method measures alcohol concentration by measuring the amount of current generated when an alcohol component contacts a platinum anode. This method offers excellent accuracy and allows for vacations, and is currently used by police and others. However, it is sensitive to external temperature and operates only within a certain temperature range. Furthermore, it is not as inexpensive as semiconductor methods.

Furthermore, the IR method measures alcohol concentration by measuring the amount of infrared radiation absorbed by alcohol. It offers excellent accuracy and is less sensitive to external influences, making it widely used for research. However, it is relatively expensive and difficult to transport.

The traditional sobriety measurement devices mentioned above have limitations in their use by multiple people, and without coercive means, measurements cannot be taken without the subject's cooperation.

In addition, for example, in the case of the traditional electrochemical method, the subject directly contacts the measuring device and measures alcohol from the exhaled breath. However, there was a problem that it was difficult to obtain cooperation in the measurement due to the negative perception of the subject due to concerns about infection with COVID-19, etc.

To solve the problems of the above traditional method, a method of measuring alcohol consumption by contacting the sensor with the human breath was researched and developed. However, this method also had the problem of requiring the subject's cooperation and taking a relatively long measurement time.

### [Detailed Description of the Invention]

### [Technical Problem]

The present disclosure provides a one-touch alcohol measurement method and device using an optical method.

### [Technical Solution]

A device for detecting ethanol content in blood, according to at least one embodiment of the present disclosure, may include: a first sensor that irradiates a plurality of lights with different wavelengths to blood vessels of a target user; a light receiving unit that receives the light irradiated to the blood vessels by the first sensor; and a processor that acquires first data based on the irradiation of light of the first wavelength and second data based on the irradiation of light of the second wavelength, corrects the second data based on the first data, and detects ethanol content data in blood based on the corrected second data.

According to at least one embodiment of the present disclosure, the device may further include: a second sensor that optically modulates light of each wavelength irradiated to the blood vessels.

According to at least one embodiment of the various embodiments of the present disclosure, the first sensor may be an optical sensor, the optical sensor may include an IR sensor, and the IR sensor may include an NIR sensor.

According to at least one embodiment of the various embodiments of the present disclosure, the second sensor may include an ultrasonic sensor.

According to at least one embodiment of the various embodiments of the present disclosure, the second sensor may use a method of indirectly modulating a signal using a refractive index of a material.

According to at least one embodiment of the various embodiments of the present disclosure, the processor may obtain a first waveform by each light source of the first sensor and a second waveform modulated by a refractive index change due to a medium change occurring at a focusing point of the second sensor in the blood, and fuse the second waveform to the first waveform as a sideband.

According to at least one embodiment of the various embodiments of the present disclosure, the first sensor may irradiate a blood vessel with light of a first wavelength, which transmits water and ethanol in the blood and has a wavelength that absorbs and reflects non-water components of the blood tissue component.

According to at least one embodiment of the various embodiments of the present disclosure, the first sensor may irradiate a blood vessel with light of a second wavelength, which transmits water in the blood and has a wavelength that reflects non-water components of the ethanol and blood tissue component.

According to at least one embodiment of the various embodiments of the present disclosure, the first data may include data measuring the influence of non-water components of the blood tissue component upon irradiating the blood vessel with light of the first wavelength.

According to at least one embodiment of the various embodiments of the present disclosure, the second data may include data measuring the influence of non-water components of the ethanol and blood tissue component.

According to at least one embodiment of the various embodiments of the present disclosure, the light of the first wavelength may be infrared light having a wavelength that transmits water and ethanol in the blood and absorbs and reflects non-water components of the blood tissue components.

According to at least one embodiment of the various embodiments of the present disclosure, the light of the first wavelength may have a wavelength of 700 to 950 nm.

According to at least one embodiment of the various embodiments of the present disclosure, the light of the second wavelength may be infrared light having a wavelength that transmits water contained in the blood and reflects non-water components of ethanol and the blood tissue components.

According to at least one embodiment of the various embodiments of the present disclosure, the light of the second wavelength may have a wavelength of 1100 to 1300 nm.

A method for detecting ethanol content in blood using light in an electronic device according to at least one of various embodiments of the present disclosure may include: irradiating the blood with a plurality of lights having different wavelengths; acquiring first data based on irradiation of light with a first wavelength among the plurality of lights and second data based on irradiation of light with a second wavelength; correcting the acquired second data based on the acquired first data; and detecting the ethanol content data in the blood based on the corrected second data.

### [Effects of the Invention]

According to at least one of various embodiments of the present disclosure, the method can conveniently extract alcohol content in blood without user discomfort.

According to at least one of various embodiments of the present disclosure, the method can more accurately extract alcohol content in the blood of a user than in the prior art.

### [Brief Description of Drawings]

FIG. 1 is a schematic diagram of an alcohol measurement system according to one embodiment of the present disclosure.
FIG. 2 is a diagram illustrating an electronic device according to an embodiment of the present disclosure.
FIG. 3 is a block diagram of the driving unit of the electronic device illustrated in FIG. 1.
FIG. 4 is a diagram illustrating a sensor for sensing ethanol components in the blood of a target user according to an embodiment of the present disclosure.
FIGS. 5 and 6 are diagrams illustrating data detection using an optical method according to an embodiment of the present disclosure.
FIGS. 7 and 8 are diagrams illustrating an optical sensing method according to an embodiment of the present disclosure.
FIGS. 9 to 11 are diagrams illustrating an optical modulation method related to the present disclosure.
FIG. 12 is a flowchart illustrating a method for detecting ethanol in blood using light according to an embodiment of the present disclosure.

### [Best Mode]

Hereinafter, embodiments related to the present disclosure will be described in more detail with reference to the drawings. The suffixes "module" and "unit" used in the following description for components are assigned or used interchangeably solely for the convenience of writing the specification, and do not inherently have distinct meanings or roles.

Software or hardware, such as programs, including methods for measuring alcohol intake according to various embodiments of the present disclosure may be embedded or implemented in vehicles, wearable devices such as smartwatches, and the like. For convenience of explanation, the methods and devices for measuring alcohol intake according to the present disclosure will be described below as being embedded or implemented in vehicles, but are not limited thereto.

Hereinafter, various embodiments of methods and devices for measuring alcohol intake according to the present disclosure will be described with reference to the attached drawings.

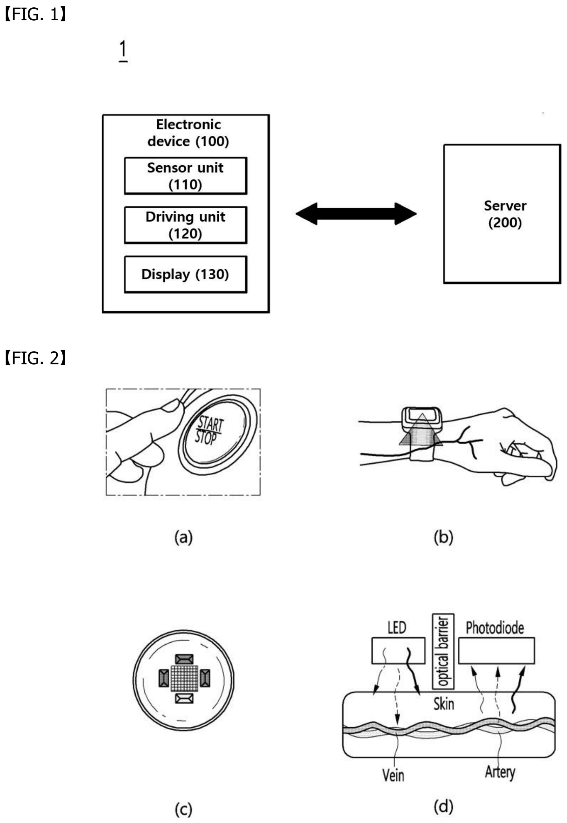

FIG. 1 is a schematic diagram of a system 1 for measuring alcohol intake according to one embodiment of the present disclosure.

FIG. 2 is a diagram illustrating an electronic device 100 according to an embodiment of the present disclosure.

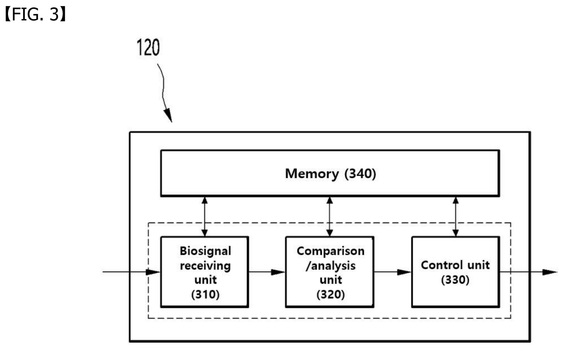

FIG. 3 is a block diagram of the driving unit 120 of the electronic device illustrated in FIG. 1.

Referring to FIG. 1, the alcohol measurement system 1 may be configured to include an electronic device 100 and a server 200.

The electronic device 100 may include a sensor unit 110, a driving unit 120, a display 130, and the like.

The electronic device 100 may be implemented in a vehicle-related configuration as illustrated in (a) of FIG. 2 (e.g., a start button provided in a vehicle, a button on a smart key for remotely controlling the vehicle, etc.), a smartwatch as illustrated in (b) of FIG. 2, or, although not illustrated, a smartphone. However, the present invention is not limited thereto, and for convenience of explanation, the electronic device 100 will be described below using a vehicle as an example.

The sensor unit 110 may include at least one sensor, sensor array, or combination thereof provided or mounted on the electronic device 100. The sensor unit 110 may, for example, sense and acquire data for measuring whether a target user within the electronic device 100 is intoxicated. The data for measuring intoxication may include data regarding ethanol (alcohol) components in blood. The sensor unit 110 may further sense additional data in addition to the aforementioned ethanol components.

Referring to (c) of FIG. 2, the sensor unit 110 is illustrated with three sensors and a light receiver for convenience of sensing the aforementioned data. The light receiver is configured to receive signals transmitted by the sensors, and is, for example, a photodiode, but is not limited thereto.

As described above, the present disclosure measures the ethanol component (e.g., ethanol concentration or amount) contained in the blood of a subject user, and determines whether the subject user is drinking based on the measured ethanol component. Meanwhile, the present disclosure includes even the driving control of an electronic device 100 based on the result of determining whether the subject user is drinking. The details of the driving control may vary depending on the type of electronic device 100, etc.

Meanwhile, in the present disclosure, when determining whether or not a subject is drinking based on the ethanol component in the blood, the criteria for the determination may refer to relevant laws, public institutions, or other arbitrarily set values. Accordingly, the criteria for the determination may be variously defined depending on region, location, country, etc.

The present disclosure uses an optical method as an example to acquire data on the ethanol component in blood. Referring to (d) of FIG. 2, a method may be used in which a optical sensor (e.g., an LED) irradiates light of a predefined wavelength to a blood vessel, and a light receiving unit (photodiode) receives the light irradiated by the optical sensor. In this case, in the present disclosure, one or more optical sensors are used to irradiate at least two different wavelengths of light to each blood vessel, thereby obtaining data on ethanol components in blood. Meanwhile, the blood vessels to which multiple lights of different wavelengths are irradiated may be arteries or veins, and while it is preferable that they are irradiated to the same location, the present invention is not limited thereto. Specific details will be described later.

When the sensor unit 110 senses the biosignal of the target user and acquires data, the drive unit 120 may acquire and process the corresponding data from the sensor unit 110.

Depending on the embodiment, the role or function of the drive unit 120 may be performed by the server 200.

According to another embodiment, when the sensor unit 110 senses the bio-signal of the target user and acquires data, the drive unit 120 and the server 200 may each process the data, and the results may be compared for final processing.

According to another embodiment, when the sensor unit 110 senses the bio-signal of the target user and acquires data, some of the data may be processed by the drive unit 120, and the remainder may be processed by the server 200. The server 200 may transmit the processed data to the drive unit 120. At this time, the transmitted data may also include a control signal for the electronic device 100 by the server 200. In addition, the transmitted data may be output through the display 130.

The display 130 may include an input/output interface such as a display panel or a speaker, and may receive a user's input or output the aforementioned data. As mentioned above, the display panel may be a touch panel, and may be any one of an LCD, an LED, an OLED, etc. However, this is not limited thereto. The display 130 may not be an essential component. For example, the display may represent the display of a terminal possessed by the target user or the display of a pre-registered terminal.

Referring to FIGS. 1 and 3, the drive unit 120 may be configured to include a processor and a memory 340.

The memory 340 need not be implemented within the drive unit 120 and may be implemented externally or remotely. If remotely located, the memory 340 may be implemented in the form of a database (DB) server or in a form linked thereto. For convenience, the following description will be given as an example of a memory provided within the drive unit 120.

The memory 340 may store various data generated or acquired from the electronic device 100. The memory 340 may store various data related to determining whether a user is intoxicated, for example, various reference data, in connection with the present disclosure. The memory 340 may store various programs, applications, software, artificial intelligence engines, etc. for determining whether a user is intoxicated according to the present disclosure.

The processor may be configured to include a biosignal receiving unit 310, a comparison/analysis/processing unit 320, a control unit 330, etc.

The biosignal receiving unit 310 may receive data sensed by the sensing unit 110. The biosignal receiving unit 310 may extract biosignals related to determining whether a user is intoxicated according to the present disclosure from the data received by the sensing unit 110.

The comparison/analysis processing unit 320 may extract data necessary for determining whether a user is intoxicated, included in the extracted biosignals of the user, and corresponding reference data stored in the memory 340, and then compare and analyze the two data.

The comparative analysis processing unit 320 may output, through the display 130, data including whether the target user's blood contains ethanol, and if so, the ethanol content, or data indicating whether the user is intoxicated based on the results of the comparative analysis, or generate driving control data to control the electronic device 100 based on the determination result data.

The control unit 330 may control the overall operation of the electronic device 100, including the driving unit 120.

Meanwhile, if the electronic device 100 is a vehicle, the driving unit 120 may control vehicle operation, such as vehicle operation, based on the determination result of whether the target user is intoxicated.

That is, the driving unit 120 may determine the driver's status, including whether the driver is intoxicated, from the driver's biosignals acquired through the sensor unit 110, and may generate and transmit a vehicle control signal based on the determination result, or directly control the vehicle.

Vehicle control signals may include various control signals related to various events within the vehicle, such as control signals related to vehicle operation, control signals unrelated to vehicle operation but related to driver status, and control signals for providing information through the display 130.

Meanwhile, the server 200 may receive or collect and process data from the vehicle 100.

In particular, when data is received from the vehicle 100, the server 200 may perform at least some of the functions of the drive unit 120 of the vehicle 100.

The server 200 may process the received data from the vehicle 100 and, based on the data, return or transmit a signal containing a control command for controlling the target vehicle 100 to the vehicle 100.

Such a server 200 may, for example, be in the form of a cloud server. The server 200 may be provided by the manufacturer of the vehicle or driver monitoring device/system, but is not limited thereto.

Meanwhile, the server 200 may not be an essential component in the present disclosure.

In the present disclosure, data on the ethanol content in the blood of a target user is acquired using a sensor, and the data can be used to measure or determine whether the target user is drinking.

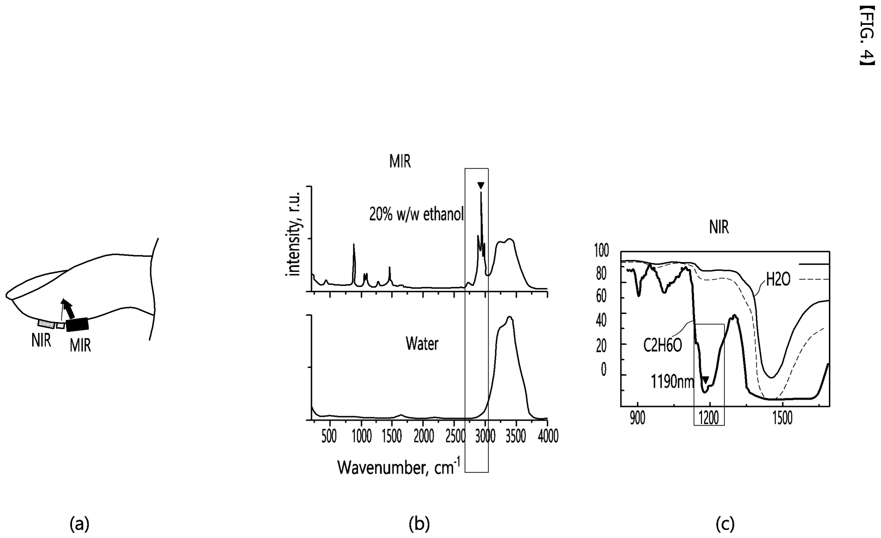

FIG. 4 is a diagram illustrating a sensor for sensing ethanol content in the blood of a target user, according to an embodiment of the present disclosure.

For convenience, in the present disclosure, when sensing ethanol content in the blood of a target user, an optical sensor, particularly an infrared (IR) sensor, may be used, depending on the finger contact area and method, as illustrated in (a) of FIG. 4. Furthermore, among the IR sensors, at least one of a mid-infrared (MIR) sensor and a near-infrared (NIR) sensor may be used.

The sensor described in the present disclosure may also include an ultrasonic sensor in addition to an optical sensor.

(b) of FIG. 4 illustrates the MIR reflectance spectra for ethanol and water in blood. This may vary depending on conditions and various factors.

(c) of FIG. 4 illustrates the NIR transmittance spectra of ethanol and water in blood. Referring to the NIR transmittance spectrum, ethanol (C2H6O) exhibits significant changes distinguishable from water (H2O) between approximately 1100 and 1300 nm (particularly, near 1190 nm).

For convenience, the following description uses NIR sensors, ultrasonic sensors, and the like as examples, but is not limited thereto. Multiple NIR sensors and ultrasonic sensors may be used in the present disclosure.

The present disclosure provides a one-touch, optical method for measuring alcohol consumption in vehicles, which allows for natural measurement without causing discomfort to the driver.

According to the present disclosure, anyone can easily measure or determine alcohol consumption with a simple, one-touch method and obtain relevant information.

However, the optical method according to the present disclosure utilizes an IR sensor, i.e., any one of near-infrared, mid-infrared, or far-infrared light can be used in terms of wavelength. While near-infrared light has the advantage of excellent skin penetration, it is difficult to obtain ethanol-specific data due to the similarities between water and ethanol. While mid-infrared light is expected to be a light source capable of capturing ethanol's specificity, its inability to penetrate the skin makes it difficult to obtain accurate or sufficient data on ethanol in blood. Finally, far-infrared light has a very long wavelength and poor penetration, limiting its utility.

Furthermore, even with optical methods, even if the light penetrates blood vessels, scattering can occur due to various substances within the blood vessels. In other words, even with optical methods, obtaining the desired data, i.e., ethanol data, within blood vessels is difficult. Therefore, a solution is needed.

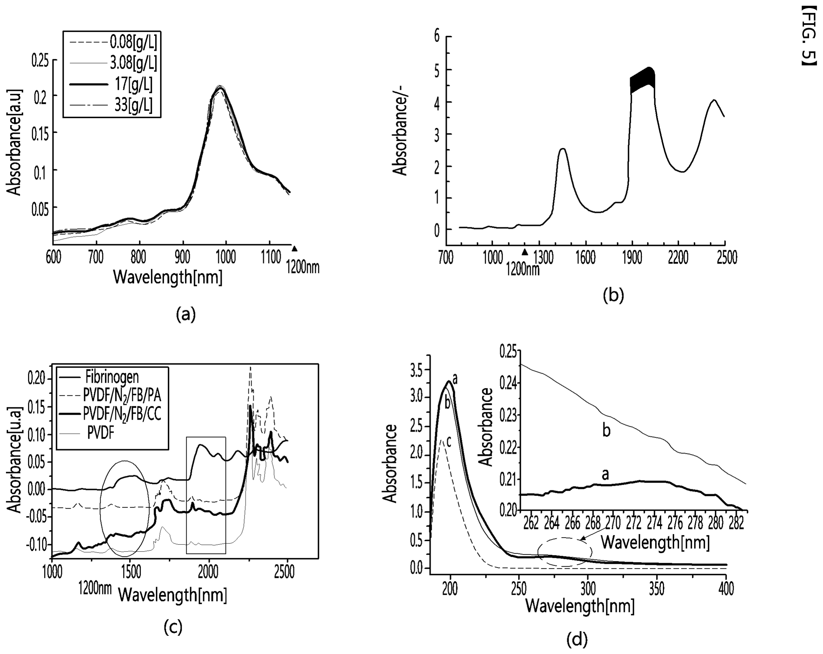

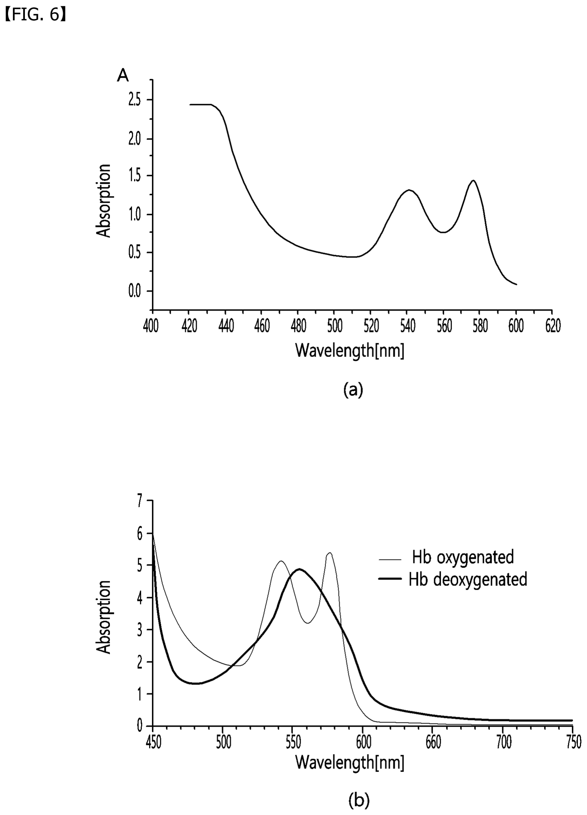

FIGS. 5 and 6 are diagrams illustrating data detection using an optical method according to an embodiment of the present disclosure.

At this time, (a) to (d) of FIG. 5 and (a) to (b) of FIG. 6 illustrate spectra according to human tissues (including blood) and components and wavelengths that should be considered to identify ethanol singularities.

Meanwhile, (c) of FIG. 4 illustrates a comparative graph of water (H2O) and ethanol (C2H6O). Referring to (c) of FIG. 4, a singularity of ethanol can be identified relative to water around 1200 nm (e.g., 1190 nm).

Meanwhile, in (a) to (d) of FIG. 5, the spectra of four types of proteins, namely albumins, globulins, fibrinogen, and prothrombin, are shown, respectively. In (a) of FIG. 6, the spectra of red blood cells (RBCs) are shown, and in (b) of FIG. 6, the spectra of hemoglobin (HB) are shown.

Referring to the spectra shown in (a) to (d) of FIG. 5, it can be seen that water and ethanol exhibited transmittable characteristics between approximately 600 and 1100 nm, whereas proteins and hemoglobin (HB) exhibited absorption or reflection characteristics.

Meanwhile, referring to the spectra shown in (a) to (d) of FIG. 5 and (a) to (b) of FIG. 6, it can be seen that water exhibits a transmittable characteristic between approximately 1100 and 1200 nm, while ethanol, protein, and hemoglobin (HB) exhibit absorption or reflection characteristics.

Using the above characteristics, we aim to separate light scattered by light irradiation from light containing valid data, thereby securing data on ethanol. This will be described in detail below.

In the present disclosure, the desired data can be secured despite scattering using the aforementioned NIR light source. In another embodiment, a light modulation method utilizing ultrasound (microwaves) can be used to improve accuracy. However, the present disclosure is not limited thereto.

Meanwhile, the NIR optical method according to the present disclosure can separate red blood cells (HB), protein, and ethanol from blood. At this time, a reflection type may be used for the separation. Conversely, a transmission type may be used to separate water and ethanol from blood. However, this is not limited thereto.

Subsequently, ethanol data may be acquired through final correction using previously measured red blood cell (HB) and protein reflection ratio values. In other words, in the present disclosure, when only obtaining data on ethanol is performed, water, protein, red blood cells (erythrocytes), and other components constituting blood may be interfering factors. Additionally, at least one of the internal skin components may also act as an interfering factor.

In the present disclosure, data on ethanol components in blood is acquired using an optical method, utilizing light of multiple wavelengths. In this way, by irradiating blood vessels with light of various wavelengths, data on reflected and transmitted components can be acquired, and from this, the desired data, i.e., ethanol component data, can be acquired.

In the present disclosure, light of a first wavelength, which transmits ethanol and reflects proteins, red blood cells, etc., is irradiated onto blood vessels, and the influence or ratio of proteins, red blood cells, etc. can be measured based on the light of the first wavelength. This can be conveniently referred to as a primary measurement, but is not limited thereto.

The first wavelength used in the primary measurement may be, for example, approximately 950 nm or less (e.g., 600 to 800 nm), but is not limited thereto.

Meanwhile, light of a second wavelength, which reflects ethanol and transmits water, can be used to measure the influence or ratio of ethanol. This can be referred to as a secondary measurement, in contrast to the primary measurement described above.

The second wavelength used in the secondary measurement may be, for example, approximately 1200-1300 nm, but is not limited thereto.

However, the order of the first and second measurements may be reversed, as they are named as the first measurement and the second measurement, respectively.

Based on the data acquired through the first and second measurements, the driving unit 120 can ensure that the actual value is obtained by reflecting the influence of ethanol, proteins, red blood cells, etc.

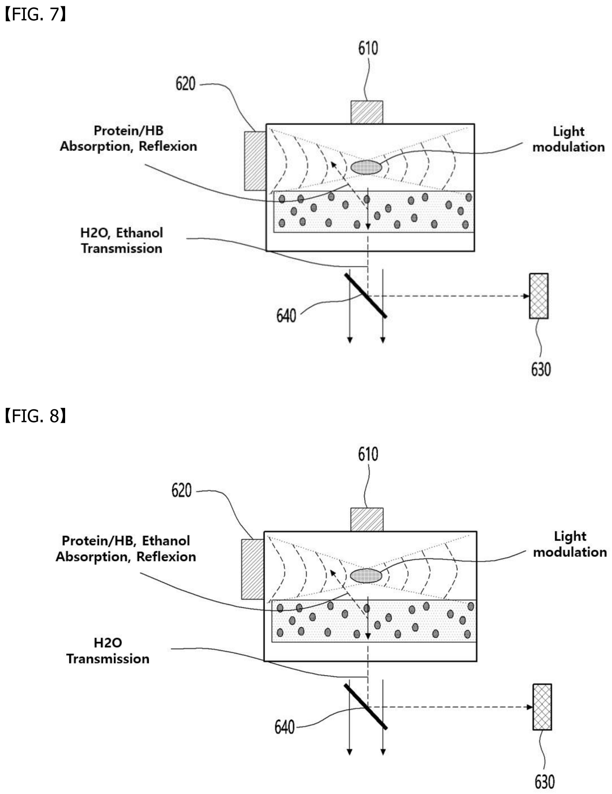

FIGS. 7 and 8 are diagrams illustrating an optical sensing method according to an embodiment of the present disclosure.

FIG. 7 can illustrate the optical sensing method for the first measurement described above.

Referring to FIG. 7, for the first measurement, the device may include a first sensor (LED) 610, a second sensor 620, a light receiving unit 630, etc.

In this case, the device may further include a mirror 640. Meanwhile, the mirror 640 may be, but is not limited to, a dichroic mirror.

The first sensor (LED) 610 may irradiate light of a first wavelength into the blood. At this time, the first wavelength may be approximately 950 nm or less.

As described above, the light of the first wavelength irradiated into the blood may, nevertheless, be scattered, absorbed, reflected, etc. before passing through the skin and reaching the blood.

The second sensor 620 may modulate the light irradiated into the blood from the first sensor 610 using ultrasound. Therefore, the second sensor 620 may be an ultrasonic sensor.

Referring to FIG. 7, according to the light of the first wavelength irradiated to the blood by the first sensor 610, water and ethanol components can be transmitted and received by the light receiving unit 630 through the dichroic mirror 640. At this time, the light receiving unit 630 may be, for example, a photodiode, but is not limited thereto.

On the other hand, according to the light of the first wavelength irradiated to the blood by the first sensor 610, components such as proteins and hemoglobin (HB) are absorbed or reflected. By irradiating the second sensor 620 with ultrasound to modulate the light, data on the components such as proteins and hemoglobin (HB) that are absorbed or reflected can be obtained. The data on the components obtained in this way can be converted into data on the influence of the corresponding components.

According to FIG. 7, since both water and ethanol are still permeable, the first measurement result alone is insufficient to derive data on only the ethanol component in the blood of the target user. Therefore, data on only the ethanol component in the user's blood can be extracted through a second measurement.

Next, FIG. 8 can illustrate the optical sensing method for the aforementioned secondary measurement.

Referring to FIG. 8, the configuration of the device for secondary measurement may be identical to the configuration for primary measurement of FIG. 7. Accordingly, reference numerals in the drawing may be indicated with the same numbering as in FIG. 7.

Referring to FIG. 8, the device for secondary measurement may be implemented, similar to FIG. 6, including a first sensor 610, a second sensor 620, a light receiving unit 630, and a mirror 640. However, according to the present disclosure, the configuration of the device for secondary measurement may differ from that of FIG. 7.

However, the first sensor (LED) 610 may irradiate light of a second wavelength into the blood. At this time, the second wavelength may be a value between approximately 1200-1300 nm, but is not limited thereto.

Light of the second wavelength irradiated to the blood vessels due to blood flow may also undergo scattering, absorption, reflection, etc. before passing through the skin and reaching the blood, just like the light of the first wavelength described above.

The second sensor 620 may modulate the light irradiated to the blood from the first sensor 610 using ultrasound.

Referring to FIG. 8, light of the second wavelength irradiated to the blood by the first sensor 610 may only pass through water and be received by the light receiving unit 630 via the dichroic mirror 640.

On the other hand, according to the light of the second wavelength irradiated to the blood by the first sensor 610, not only components such as proteins and hemoglobin (HB) but also ethanol are absorbed or reflected. This can be achieved by irradiating ultrasonic waves from the second sensor 620 to modulate the light, thereby obtaining data on the absorbed or reflected components such as proteins and hemoglobin (HB) and the ethanol component. The data of the components thus obtained can be converted into influence data of the corresponding components.

In FIG. 7, water and ethanol were transmitted, and proteins and hemoglobin (HB) were absorbed/reflected, according to the irradiation of light of the first wavelength, and in FIG. 8, only water was transmitted, and components such as ethanol, proteins and hemoglobin (HB) were absorbed/reflected, according to the irradiation of light of the second wavelength.

Therefore, by referring to the contents of FIGS. 7 and 8, and by correcting the absorption/reflection component data obtained in FIG. 8 with reference to the absorption/reflection component data obtained in FIG. 7, data on only the ethanol component in blood can be extracted.

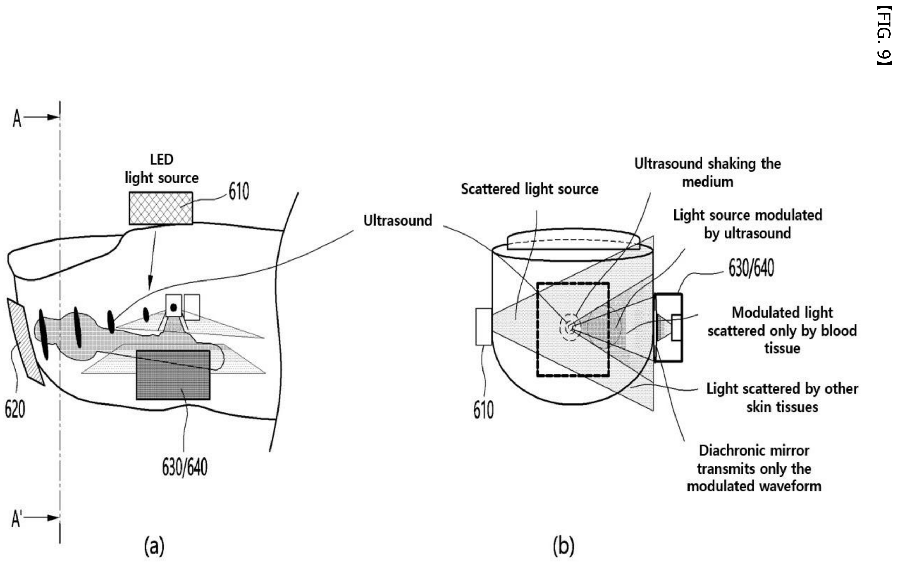

FIGS. 9 to 11 are diagrams illustrating an optical modulation method using ultrasound according to an embodiment of the present disclosure.

(a) of FIG. 9 illustrates a side view of a user's body part, for example, a finger.

For example, if an electronic device 100 is provided with a groove (not shown) into which a finger can enter and exit, the groove may include the first sensor 610 and the second sensor 620 described above.

Based on the finger entry direction, a first sensor (or first sensor array) 610 is provided at the upper portion of the groove, and can irradiate light of the first wavelength and the second wavelength to the inside of the finger, i.e., the blood, as illustrated in (a) of FIG. 9.

Alternatively, the first sensor (or sensor array) 610 may be formed or arranged on a side, rather than the upper portion, of the groove.

Meanwhile, a second sensor 620 may be provided, for example, in an area of the groove corresponding to the front surface of the finger entry direction, as illustrated in (a) of FIG. 9.

Referring to (a) of FIG. 9, for example, the entry directions of the light of the first wavelength and/or the second wavelength and the ultrasound into the finger may be perpendicular to each other or at least partially intersect. Accordingly, the first sensor 610 and the second sensor 620 may be arranged accordingly.

For convenience of explanation, the present disclosure discloses only one optical sensor and one ultrasonic sensor, but the present disclosure is not limited thereto, and each may be provided in multiple numbers.

(b) of FIG. 9 illustrates a view taken along line A-A' in the drawing illustrated in (a) of FIG. 9, i.e., a view viewed from the direction facing a finger entering the groove.

Meanwhile, (a) of FIG. 9 illustrates a case where the first sensor 610 is formed or arranged on the upper portion of the groove, while (b) of FIG. 9 illustrates a case where the first sensor 610 is formed or arranged on the side of the groove.

Referring to (b) of FIG. 9, the first sensor 610 is formed (or arranged) on the left side of the finger, so that light of the first wavelength and the second wavelength can be irradiated toward the inside of the finger.

At this time, the second sensor 620 formed or arranged in a direction facing or corresponding to the fingertip can irradiate ultrasound toward the finger.

As illustrated in (b) of FIG. 9, light irradiated by the first sensor 610 is modulated by the irradiated ultrasound, and the mirror or light-receiving unit formed or arranged opposite, i.e., facing, the first sensor 610 can only receive light that has been modulated by blood tissue (i.e., light of the first or second wavelength modulated by ultrasound and then scattered by blood tissue).

Therefore, in this case, light scattered by skin tissue other than the aforementioned blood tissue can be controlled so as not to be input to the mirror 630 or light-receiving unit.

To this end, the electronic device 100 can determine the direction of entry and the arrangement of each sensor, taking into account each wavelength and the wavelength of the ultrasound.

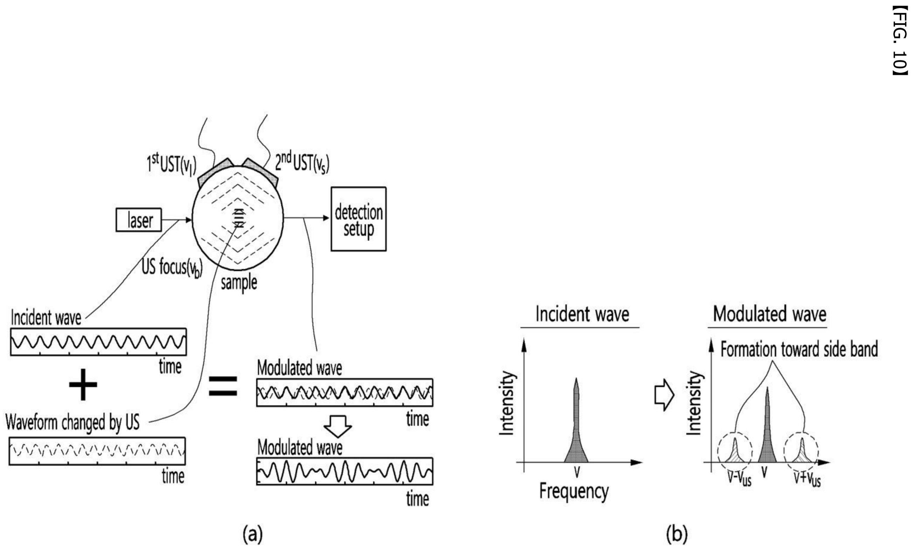

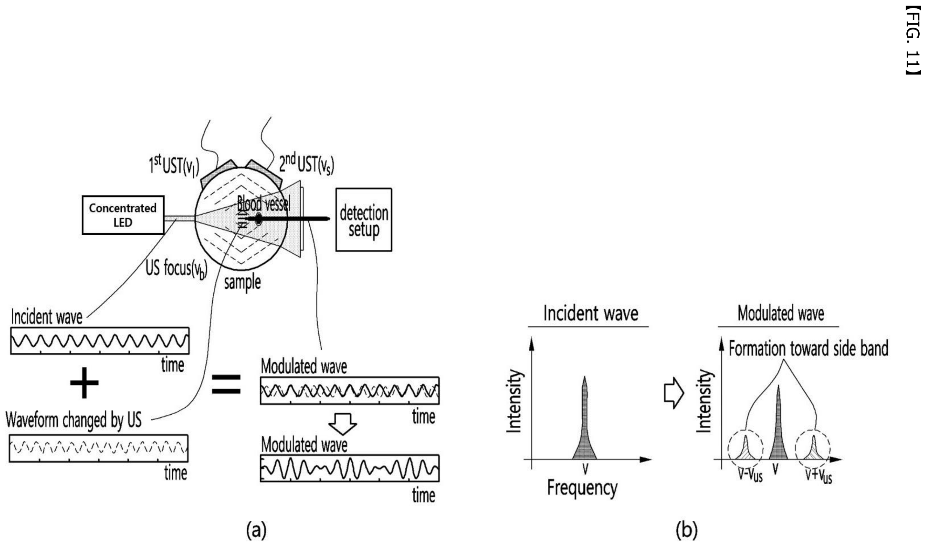

FIGS. 10 and 11 illustrate, in particular, the optical modulation method related to the present disclosure.

First, referring to FIG. 9, modulation is performed to convert an electrical signal into an optical signal and transmit and receive at the speed of light. This modulation may include direct modulation and indirect modulation.

The present disclosure may utilize, for example, indirect modulation. However, the present disclosure is not limited thereto.

That is, the present disclosure may utilize indirect modulation, i.e., a method of modulating a signal using the refractive index of a material.

For this purpose, as described above, the present disclosure may utilize ultrasound to induce a change in the refractive index of a material.

(a) of FIG. 10 illustrates Ultra-sounds Optical Tomography (UOT) to illustrate an embodiment of the present disclosure.

Referring to (a) of FIG. 10, ultrasound may have the property of being a material wave that propagates through a medium. Furthermore, changes in the refractive index due to changes in the medium may occur at the point where the ultrasound is focused.

Referring to (a) of FIG. 10, for example, when a laser source irradiates a target with a laser beam (incident wave), a modulated wave can be detected in the detection setup due to the waveform changed by ultrasound focusing (US).

In this case, as illustrated in (b) of FIG. 10, the modulated waveform can be fused into sidebands.

Applying the principle described in FIG. 10 to the present disclosure, an implementation can be achieved, as illustrated in FIG. 11.

(a) of FIG. 11 illustrates an Ultra-Sound Optical System (UOS).

In this case, (a) of FIG. 11 is equipped with an LED light source, i.e., a first sensor 610 capable of irradiating light of first and second wavelengths, instead of the laser light source disclosed in (a) of FIG. 10.

Therefore, referring to the system illustrated in (a) of FIG. 11, the wavelength of light input by the first sensor 610 and the wavelength modified by the focused ultrasound are combined to form a modulated waveform that can be received by the detection setup.

The intensity of light emitted by the system illustrated in (a) of FIG. 11 is depicted in (b) of FIG. 11, similar to the graph illustrated in (b) of FIG. 9.

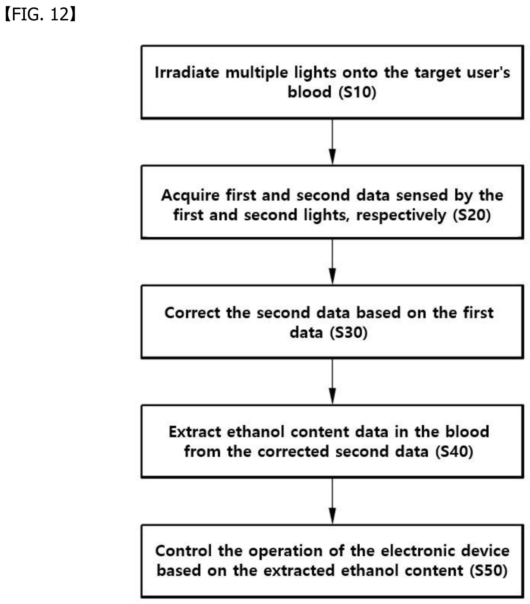

FIG. 12 is a flowchart illustrating a method for detecting ethanol in blood using light, according to an embodiment of the present disclosure.

A method for detecting ethanol in blood using multiple lights in an electronic device 100 is described. While FIG. 12 illustrates the electronic device 100, the present disclosure is not limited thereto.

In step S10, the electronic device 100 may irradiate multiple lights onto the human body of a target user, i.e., onto the blood. For convenience of explanation, two different wavelengths of light may be irradiated onto the blood of the same user.

In this case, the body parts to which the two different wavelengths of light are irradiated may be the same, but the present disclosure is not limited thereto.

In step S20, the electronic device 100 may obtain first data based on the irradiation of the first light and second data based on the irradiation of the second light.

In step S30, the electronic device 100 may correct the second data based on the first data acquired in step S20.

In step S40, the electronic device 100 may extract blood ethanol content data of the target user based on the corrected second data.

In step S50, the operation of the electronic device 100 may be controlled based on the extracted blood ethanol content data of the target user. For example, as described above, if the electronic device 100 is a vehicle, the electronic device 100 may be controlled to ignore a request from the target user related to vehicle operation or to restrict vehicle operation in whole or in part despite the request.

Generally, when the electronic device 100 of the present disclosure is a vehicle, it may be desirable to place it on the start button of the driver's seat, as illustrated in FIG. 2. However, to prevent misuse by someone other than the driver (e.g., someone who has not been drinking), the first step is to place it on the start button. However, to prevent misuse by someone other than the driver, theft is prevented.

For convenience of explanation, the present disclosure has been described using a case where a breathalyzer and method are included in a vehicle. However, as described above, the present disclosure can be applied to various electronic devices or systems, such as smartwatches.

Even if not specifically mentioned, the order of at least some of the operations disclosed in the present disclosure may be performed simultaneously, in a different order than the previously described order, or some may be omitted or added.

According to one embodiment of the present disclosure, the above-described method can be implemented as processor-readable code on a program-recorded medium. Examples of processor-readable media include ROM, RAM, CD-ROM, magnetic tape, floppy disk, and optical data storage devices.

The display device described above is not limited to the configurations and methods of the embodiments described above. Furthermore, the embodiments may be configured by selectively combining all or part of the embodiments to allow for various modifications.

### [Industrial Applicability]

The present disclosure relates to a device and method for extracting data on ethanol components in blood using an optical method and measuring or determining whether a person is intoxicated with a single touch based on the extracted ethanol component data. This device has potential for application in various industrial fields, such as vehicles and wearable devices, and thus has industrial applicability.

## Claims

1. An apparatus for detecting an ethanol component in blood, comprising:
a first sensor configured to irradiate a plurality of lights having different wavelengths respectively onto a blood vessel of a target user;
a light receiving unit configured to receive the lights respectively irradiated onto the blood vessel by the first sensor; and
a processor configured to acquire first data corresponding to irradiation of a first wavelength of light and second data corresponding to irradiation of a second wavelength of light, to correct the second data based on the first data, and to detect ethanol component data in the blood based on the corrected second data.

2. The apparatus according to claim 1, further comprising a second sensor configured to optically modulate each wavelength of light irradiated onto the blood vessel.

3. The apparatus according to claim 2, wherein the first sensor is an optical sensor, the optical sensor includes an infrared (IR) sensor, and the IR sensor includes a near-infrared (NIR) sensor.

4. The apparatus according to claim 2, wherein the second sensor includes an ultrasonic sensor.

5. The apparatus according to claim 4, wherein the second sensor operates in a manner that indirectly modulates a signal using a refractive index of a substance.

6. The apparatus according to claim 5, wherein the processor is configured to acquire a first waveform corresponding to each light source of the first sensor and a second waveform modulated according to a refractive index variation caused by a medium fluctuation at a focusing point of the second sensor in the blood, and to merge the second waveform as a sideband with the first waveform.

7. The apparatus according to claim 1, wherein the first sensor irradiates, onto the blood vessel, light of a first wavelength that transmits water and ethanol in the blood but is absorbed and reflected by non-aqueous tissue components of the blood.

8. The apparatus according to claim 7, wherein the first sensor irradiates, onto the blood vessel, light of a second wavelength that transmits water in the blood but is reflected by ethanol and non-aqueous tissue components of the blood.

9. The apparatus according to claim 8, wherein the first data includes influence measurement data of non-aqueous tissue components of the blood resulting from irradiation of the first wavelength of light onto the blood vessel.

10. The apparatus according to claim 8, wherein the second data includes influence measurement data of ethanol and non-aqueous tissue components of the blood.

11. The apparatus according to claim 1, wherein the light of the first wavelength is infrared light that transmits water and ethanol in the blood but is absorbed and reflected by non-aqueous tissue components of the blood.

12. The apparatus according to claim 11, wherein the light of the first wavelength has a wavelength range of 700 nm to 950 nm.

13. The apparatus according to claim 1, wherein the light of the second wavelength is infrared light that transmits water in the blood but is reflected by ethanol and non-aqueous tissue components of the blood.

14. The apparatus according to claim 13, wherein the light of the second wavelength has a wavelength range of 1100 nm to 1300 nm.

15. A method for detecting an ethanol component in blood using light in an electronic device, comprising:
irradiating a plurality of lights having different wavelengths respectively onto the blood;
acquiring first data corresponding to irradiation of a first wavelength of light and second data corresponding to irradiation of a second wavelength of light;
correcting the acquired second data based on the acquired first data; and
detecting ethanol component data in the blood based on the corrected second data.
